# EUROPEAN PATENT SPECIFICATION

(11) **EP 2 191 865 B1**
(45) Date of publication and mention of the grant of the patent: **23.10.2013**
(21) Application number: 08020521.4
(22) Date of filing: 26.11.2008
(51) Int. Cl.: A61Q 5/04, A61Q 5/10, A61K 8/00

(54) **Process for colouring and straightening keratin fibres**
Verfahren zur Färbung und dauerhaften Glättung von Keratinfasern
Procédé pour colorer et lisser des fibres de kératine

(43) Date of publication of application: 02.06.2010
(73) Proprietor: Kao Germany GmbH, 64297 Darmstadt (DE)
(72) Inventor: Nöcker, Bernd, Dr., Tokyo 105-0011 (JP); Wood, Jonathan, Dr., 69469 Weinheim (DE); Hullmann, Alexandra, Dr., 64331 Weiterstadt (DE); Kubatz, Horst, 64665 Alsbach-Hähnlein (DE); Weißbach, Kornelia, 65812 Bad Soden (DE)
(74) Representative: Grit, Mustafa

(56) References cited:
- EP-A- 0 083 095
- EP-A- 0 260 716
- EP-A- 1 655 056
- US-A1- 2005 076 459

## Description

The present invention is related to a process for colouring and straightening keratin fibres especially human hair in a single process.

Oxidative colouring hair is a common practice in hair dressing area. Straightening is also a common process and especially straightening naturally curled and/or frizzy hair is used in order to achieve a smoother hair surface.

Currently, these two chemical alteration processes of keratin fibres especially human hair are carried out separately in two independent processes in order to secure optimal and long lasting colouration and straightening. In practice a person willing to have both chemical services is, therefore, asked to visit the hair dressing salon twice within a short period of time. In the first visit, hair is straightened and after a week of two or three hair is oxdatively coloured. This brings about first of all economical problems and secondly it is very much time intensive.

There are a few attempts made to carry out two chemical services in a single process, in the same hair dresser visit. For example EP 1655056 A1 discloses a process for colouring and curling hair wherein oxidative colouration is carried out first and the hair is treated with an acidic treatment and after rinsing off the acidic treatment and rolling up the hair onto curlers, a keratin reducing agent is applied onto hair and processed and finally hair is fixed with an oxidizing agent and treated with an acidic treatment again, if necessary. It has been observed that with the suggested method hair is not curled very effectively and also colours so obtained lack intensity, shine and especially durability. In the publication, nothing is disclosed on straightening keratin fibres, especially hair.

Other than the above suggested process, it has been a long hair dressing practice to use the fixing stage of a straightening process at the same time for colouring hair. It should be noted that a straightening hair involves two steps. In the first step the disulfide bonds are broken by using a reducing agent in an alkaline medium and in the second stage the broken disulfide bonds are recovered using an oxidizing agent. Oxidizing agent used in a straightening process usually comprises low level of oxidizing agent and more importantly does not have a strong alkaline pH, rather has acidic to slight alkaline pH where enough oxygen is provided to recover disulfide bonds. This is, however, quite different condition from oxidative colouring hair since there is a need for neutral to strong alkaline conditions and high concentration of oxidizing agent for securing intensive, homogeneous and long lasting colours.

The present invention starts from the objective in achieving excellently intensive, shiny colours and at the same time excellent straightening of keratin fibres without damaging keratin fibres excessively.

It has surprisingly been found out when keratin fibres especially human hair is coloured and straightened according to a process described below, colour intensity, brilliance and durability is excellent and hair is excellently straightened. It has further been observed that hair is less damaged at the end of inventive process of the present invention, feels natural and soft and has further its natural properties such as elasticity so that hair can move freely with the movement of person's head.

A novel process for colouring and straightening hair in a single process according to the present invention includes the following steps:
a- an aqueous oxidative colouring composition based on at least one oxidative dye precursor and optionally at least one coupling substance but not comprising any direct dye is mixed with a composition comprising at least one oxidizing agent and applied onto hair and rinsed off from hair with water after processing 5 to 45 min at a temperature of 20 to 45°C, optionally shampooed,
b- towel dried,
c- an aqueous composition comprising at least one reducing agent is applied onto hair and rinsed off from hair with water after a processing time of 5 to 60 min at ambient temperature wherein hair is continuously combed through during processing with reducing agent and towel dried,
d- optionally hair is treated with a composition comprising salt and optionally rinsed off,
e- optionally a composition comprising at least one oxidizing agent is applied onto hair processed for 2 to 15 min and optionally rinsed off from hair with water and optionally towel dried, and
f- an aqueous oxidative colouring composition based on at least one oxidative dye precursor and optionally at least one coupling substance is mixed with a composition comprising at least one oxidizing agent and applied onto hair and rinsed off from hair with water after processing 5 to 45 min at a temperature of 20 to 45°C and towel dried and dried with a dryer or left to dry in the air.

The oxidative colouring composition used in the step "a" of the above described process comprises at least one oxidative dye precursors. In general any oxidative dye precursor is suitable for the purpose of the present invention.

Suitable examples are p-phenylenediamine, p-methylaminophenol and substituted p-phenylenediamines such as 2,5-diamino-toluene, 2-n-propyl or 2-ethyl-p-phenylenediamine, 2,6-di-methyl-p-phenylene- diamine, 2-(2,5-diaminophenyl) ethanol, 1-amino-4-bis-(2'-hydroxy-ethyl)amino- benzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, pyrazole and the derivatives thereof such as 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diamino-phenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene or the water-soluble salts thereof.

Further suitable aminopyridines are 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2(β-hydroxyethyl amino)-6-methoxy-pyridine, 2,6-dimethyl amino-5-aminopyridine, 2-di(hydroxyethyl) amino-5-aminopyridine, 2-hydroxyethyl amino-5-aminopyridine, 4-hydroxy-2,5,6-triaminopyrimidine and/or the water-soluble salts thereof.

Further, Indole and indoline derivatives can as well be contained in the colouring composition of the present invention. Examples to those are: 6-aminoindol, 6-hydroxyindole, 1-ethyl-6-hydroxyindole, 1-methyl-4-hydroxyindol, 1-methyl-6-hydroxyindole, 2-methyl-6-hydroxyindole, 5-hydroxyindol, 4-hydroxyindol, 5,6-dihydroxyindole, 6-aminoindoline, 6-hydroxyindoline, 1-ethyl-6-hydroxyindoline, 1-methyl-4-hydroxyindoline, 1-methyl-6-hydroxyindoline, 2-methyl-6-hydroxyindoline, 5-hydroxyindoline, 4-hydroxyindoline, 5,6-dihydroxyindoline and their respective salts.

In another preferred embodiment of the present invention, aqueous oxidative colouring composition is applied onto hair in step "a" comprises at least one coupling agent. In general any coupling agent known in the art is suitable for the purpose of the present invention.

Suitable coupling agents are resorcinol, 2-methyl resorcinol, 4-chlororesorcinol, 2-amino-4-chlorophenol, 5-amino-4-methoxy-2-methylphenol, 3-amino-phenol, 1-methyl-2-hydroxy-4-aminobenzene, 3-N,N-dimethyl aminophenol, 2.6-dihydroxy-3.5-dimethoxypyridine, 5-amino-3-methylphenol, 6-amino-3-methylphenol, 3-amino-2-methylamino-6-methoxypyridine, 2-amino-3-hydroxy-pyridine, 2-dimethyl-amino-5-aminopyridine, 2,6-diaminopyridine, 1,3-diamino- benzene, 1-amino-3-(2'-hydroxyethylamino)benzene, 1-amino-3-[bis(2'-hydroxy-ethyl) amino]benzene, α -naphthol, 4,6-dichlororesorcinol, 1,3-diamino-toluene, 1-hydroxy naphthalene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof. 5-amino-2-methylphenol, 2-methyl-5-hydroxyethylaminophenol, 2,4-diamnophenoxyethanol, 2-amino-4-hydroxyethylaminoanisol, 2-methyl-5-amino-6-chlorophenol, 1,3-bis(2,4-diaminophenoxy)propane, 2-bis(2-hydroxyethyl)aminotoluene, 2-amino-5-methylphenol or the water-soluble salts thereof.

Concentration of oxidation dyes precursors and coupler is customarily in the range of 0.01 to 5% by weight calculated to total composition prior to mixing.

Aqueous oxidative colouring composition is mixed with an aqueous composition comprising at least one oxidizing agent in step "a" of the above process. As a rule, any oxidizing agent is suitable; the most preferred is hydrogen peroxide.

Concentration of hydrogen peroxide in the composition is between 2 to 12% by weight calculated to the oxidizing composition prior to mixing.

Mixing ratio of composition comprising at least one oxidative dye precursor and optionally at least one coupler with a composition comprising at least one oxidizing agent is in a weight ratio range of 3:1 to 1:3, preferably 2:1 to 1:2 and most preferably 1:1.

The aqueous oxidative colouring composition used in step "a" has a pH in the range of 5 to 12, preferably 6 to 10.5, more preferably 6.5 to 10 after mixing with an aqueous oxidizing composition and measured at room temperature. Particularly preferred pH is around neutral pH namely 6.6 to 9.5 again after mixing with an aqueous composition comprising at least one oxidizing agent.

Processing time of oxidative colouring composition in step "a" is preferably 5 to 30 min and more preferably 10 to 20 min.

The straightening compositions used in the process according to the invention comprise at least one reducing compound at a concentration of at least 0.5% by weight calculated to total composition. Preferred are thioglycolic acid and thiolactic acid as well as the salts thereof, in particular the ammonium and ethanolamine salts. Further useful thio compounds are in particular cysteine or the hydrochloride thereof, homocysteine, cysteamine, N-acetyl cysteine, thioglycerol, ethanediol monothioglycolate, 1,2-propyleneglycol monothioglycolate (see also WO-A 93/1791), 1,3-propanediol monothioglycolate or the isomer mixture resulting therefrom, 1,3-butanediol and 1,4-butanediol monothioglycolate and the isomer mixtures therefrom, polyethylene glycol, such as di-, tri- and tetraethyleneglycol monothioglycolates, glycerol monothiolactate and further thio acids and the esters thereof, as well as mixtures thereof.

The use of inorganic reducing sulfur compounds such as sodium hydrogen sulfite is basically also possible.

The total reduction agent content in the compositions according to the invention customarily amounts from 2.0 to 15 %, preferably 2.5 to 12.5% by weight, calculated to total or reducing composition.

The straightening compositions containing reducing agents used in step "c" of the above process can, if necessary, comprise alkalizing agents. Their quantity is dependent on the reducing agent and the desired pH-value of the composition. Reducing agent compositions preferably contain 0.1 % to 5%, in particular 0.5% to 2.5% by weight thereof, calculated to the total composition. Alkalizing agents preferred within the scope of the invention are ammonia, hydroxyalkyl amines such as monoethanol amine and triethanolamine, ammonium carbamate, ammonia and/or ammonium(bi)carbonate. It is desirable to adjust the pH-value between about 6.5 and 10.5, preferably about 7 to 9.5.

The viscosity best suited for the reducing compositions used in the above processes step "c" depends very much on the nature of the process. In the first process described above, wherein hair is fastened on a hard surface with aid of composition comprising at least one reducing agent, comparatively higher viscous compositions are preferred. Viscosity of such compositions are typically 5,000 mPa.s or more, preferably 7,500 mPa.s or more, more preferably 10,000 mPa.s or more measured at 20°C in a Brookfield viscosimeter with an appropriate spindle and rotation (i.e. spindle no. 5 at 5 rpm). The reducing composition described above is certainly suitable for the other two processes described above. However, for these two process viscosity is not very critical so the compositions can have lower viscosity values. In the preferred embodiment such compositions should preferably have viscosity values in the range of 500 to 10,000 mPa.s, more preferably 750 to 7,500 m.Pa.s and most preferably 1,000 to 5,000 mPa.s, measured at 20°C with Brookfield viscosimeter with an appropriate spindle and rotation (i.e. spindle no. 5 at 5 rpm).

The viscosity is adjusted by addition of the appropriate amounts of thickening agents known per se, such as cellulose derivatives. Thickening may as well be realized by formulating a composition in form of an emulsion with the use of C₁₀-C₂₂-fatty alcohols, in admixture with long mono alkyl chain quaternary ammonium surfactants.

Compositions of step "d" according to the processes disclosed above have the function of deswelling hair and therefore they comprise at least one salt preferably in a concentration range from 0.5 to 15%, more preferably 1 to 12.5% and most preferably 2 to 12.5% by weight calculated to total composition. In the process
wherein hot iron is used, the composition has at the same time the function of thermo protection and comprises at least one oil and/or oily compound.

In principal any water soluble salt is suitable for the purpose of the composition of step "d". Preferably salt is an inorganic substance. In the preferred embodiment, salts are preferably selected from salts of mono or divalent cations with mono and divalent anions. Preferred cations are sodium, calcium, potassium and magnesium and anions are chloride and sulfate. Suitable ones are such as sodium chloride, sodium sulfate, magnesium sulfate, potassium chloride, potassium sulfate, magnesium chloride, calcium chloride, ammonium salts such as ammonium chloride and ammonium sulfate. Preferred salts are sodium chloride, sodium sulfate, magnesium sulfate, potassium chloride, potassium sulfate, magnesium chloride, and calcium chloride. More preferably the salts are sodium chloride, sodium sulfate, magnesium sulfate, potassium chloride, potassium sulfate and magnesium chloride. In particular, with magnesium sulfate and sodium chloride exceptionally good results were observed.

Composition used in step "d" in combination with hot iron comprise at least one oil and/or oily compound at a concentration of 10 to 100%, preferably 15 to 90%, more preferably 20 to 80% and most preferably 25 to 70% by weight calculated to total composition.

Suitable oils are selected from natural and synthetic oily. Natural oils are vegetable triglycerides such as avocado oil, olive oil, almond oil, peach oil, passiflora oil, black cumin oil, borage oils, evening primrose oil, grapeseed oil, hempseed oil, kukui nut oil, rosehip oil, safflower oil, walnut oil and weatgerm oil. Natural oil is preferably used at a concentration of 0.1 to 5% by weight calculated to total composition.

Among synthetic oils, fatty acid fatty alcohol esters are suitable according to the formula

R₇ C (O) O R₈

wherein R₇ is saturated or unsaturated, branched or straight alkyl chain with 7-23 C atoms and R₈ is saturated or unsaturated, branched or straight alkyl chain with 1 to 24 C atoms. Preferably, R₈ is 1 to 12, more preferably 1 to 8 and most preferably 1 to 4 C atoms.

Suitable examples are behenyl behenate, behenyl isostearte, butyl stearate, butyl oleate, butyl myristate,butyloctyl oleate,cetyl palmitate, cetyl myristate, cetyl oleate, cetyl caprylate, cetyl caprate, decyl oleate, decyl cocoate, decyl isostearate, ethylhexyl myristate, ethyl hexyl laurate, ethyl hexyl oleate, ethyl isostearte, ethyl laurate, ethyl linoleate, ethyl myristate, ethyl oleate, ethyl palmitate, ethylricinoleate, ethyl stearate, hexyl isostearet, hexyl laurate, hexyl myristate, hexyl stearate, hexyl decyl oleate, isobutyl laurate, isobutyl myristate, isobutyl palmitate, isobutyl stearate, isocetyl behenate, isobutyl laurate, isobutyl oleate, isobutyl stearate, isobutyl cocoate, isohexyl caprate, isopropyl palmitate, isopropyl stearate, isopropyl behenate, isopropyl laurate, isopropyl oleate, isopropyl ricinoleate and isopropyl palmitate.

Fatty acid fatty alcohol esters are present at a concentration of 0.1 to 10% by weight calculated to total composition.

Among synthetic oils silicones are the preferred ones and can be used alone or in combination with the above mention natural oils and/or fatty acid fatty alcohol esters. Silicones suitable are volatile and non-volatile ones and volatile ones are preferred and can be sued alone or in combination with one or more other type of silicones such as arylated silicones and high viscosity and/or high molecular weight silicones. Volatile silicones are dimethicones with low viscosity such as 0.65 cSt or 1 cSt, and cyclic silicones such as cycloheptasiloxane, cyclohexasiloxane, cyclopentasiloxane, cyclotetrasiloxane, cyclotrisiloxane and cyclomethicone. Nonvolatile ones are dimthicones with high viscosity commercially available from Dow Corning with the trade name DC 200 with various viscosity and as well as arylated silicones such as phenyl methicone, phenyl trimethicone, diphenyl dimethicone, diphenylsiloxy phenyl trimethicone, tetramethyl tetraphenyl trisiloxane, triphenyl trimethicone, tetramethly tetraphenyl trisiloxane and trimethyl pentaphenyl trisiloxane.

Silicones are comprises at a concentration of 10 to 100%, preferably 15 to 90%, more preferably 20 to 80% and most preferably 25 to 70% by weight calculated to total composition. In another preferred from more than 50% by weight of the above mentioned concentration must be volatile silicone.

Composition comprising at least one oxidizing agent used in step "e" of the above novel process comprises preferably hydrogen peroxide as an oxidizing agent at a concentration of preferably 1 to 5%, more preferably 2 to 3% by weight calculated to total composition. The pH of the composition is in the range of 2 to 6, preferably 2.5 to 5, more preferably 3 to 5 and most preferably 3 to 4.5 measured at room temperature.

In the novel process of the present invention, aqueous oxidative colouring compositions used in step "f" can basically be the same composition used in step "a". At this step pH of the aqueous oxidative colouring composition is in the range of 5 to 12, preferably 6 to 11 and more preferably 6.5 to 10 and most preferably 6.6 to 9.5, measured after mixing with an aqueous composition comprising at least one oxidizing agent preferably hydrogen peroxide at a concentration of 1 to 12% by weight calculated to total composition prior to mixing.

The above mentioned oxidative dye precursors and couplers are also suitable for oxidative dyeing compositions used in step "f" of the above novel process.

Furthermore, oxidative colouring composition used in step "f" of the above novel process may comprise direct dyes. Suitable direct dyes are those anionic, cationic and neutral nitro dyes. They can also be used in mixture.

Any cationic direct dye is in principal suitable for the compositions. Examples are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87.

Any anionic dye is in principal suitable for the compositions. Suitable examples are such as Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Among those, the preferred anionic dyestuffs are Acid Red 52, Acid Violet 2, Acid Red 33, Acid Orange 4, Acid Red 27 and Acid Yellow 10 and their salts. The most preferred anionic dyes are Acid Red 52, Acid Violet 2, Acid Red 33, Acid Orange 4 and Acid Yellow 10, and their salts

Neutral dyes, so called nitro dyes for shading purposes are also optionally contained in the compositions. Suitable ones are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Concentration of one or more direct dyes in total is in the range of 0.001 to 5% by weight, preferably 0.01 to 3% more preferably 0.05 to 2% by weight calculated to total composition prior to mixing with a composition comprising oxidizing agent.

Either oxidative colouring compositions used in steps "a" and "f" and/or reducing composition used in step "c" and/or the composition of step "d" and/or oxidizing composition used in steps "a" "e" and "f" can comprise the following ingredients explained in detail below, unless otherwise stated.

The above mentioned compositions, any of the composition used in the processes mentioned above, comprise surfactants selected from anionic, nonionic, cationic and amphoteric ones. Their proportion ranges from 0.05 % to 10%, in particular from 0.1 % to 5 % by weight, calculated to total composition.

Suitable anionic surfactants are especially the known alkyl ether sulfates and carboxylic acids, in particular in form of their alkali salts, as well as protein fatty acid condensates.

Suitable nonionic surfactants, which are preferred within the scope of the invention, are in particular C₈-C₁₈-fatty alcohol polyglycol ethers, fatty acid polyglycol esters, fatty acid alkanolamides, amineoxides, and especially C₈-C₁₈-alkyl polyglucosides.

Also possible is the incorporation of amphoteric surfactants, such as the known alkyl betaines, alkyl amido betaines, and alkyl amphoacetates.

Further according to a further preferred embodiment, the above mentioned compositions comprise at least one cationic surfactant according to general formula 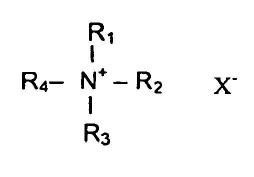 where R₁ is a saturated or unsaturated, branched or non-branched alkyl chain with 8-22 C atoms or

R₅CO NH (CH₂)ₙ

where R₅ is saturated or unsaturated, branched or non-branched alkyl chain with 7-21 C atoms and n has typical value of 0 - 4 or

R₆CO O (CH₂)ₙ

where R₆ is saturated or unsaturated, branched or non-branched alkyl chain with 7-21 C atoms and n has typical value of 0 - 4, and
R₂ is a hydrogen, saturated or unsaturated, branched or non-branched alkyl chain with 1-22 C atoms or

R₅CO NH (CH₂)ₙ

where R₅ is saturated or unsaturated, branched or non-branched alkyl chain with 7-21 C atoms and n has typical value of 0 - 4 or

R₆ CO O (CH₂)ₙ

where R₆ is saturated or unsaturated, branched or non-branched alkyl chain with 7-21 C atoms and n has typical value of 0 - 4,
and R₃ and R₄ are independent from each other H or lower alkyl chain with 1 to 4 carbon atoms, and X is chloride, bromide or methosulfate.

Concentration of cationic surfactant is in the range from 0.05 % to 5 %, preferably 0.1 % to 2.5 % by weight, calculated to total composition.

Suitable long-chain quaternary ammonium compounds which can be used alone or in admixture are in particular cetyl trimethyl ammonium chloride, dimethyl dicetyl ammonium chloride, trimethyl cetyl ammonium bromide chloride, stearyl trimethyl ammonium chloride, dimethyl stearyl benzyl ammonium chloride, benzyl tetradecyl dimethyl ammonium chloride, dimethyl dihydrogenated tallow ammonium chloride, lauryl pyridinium chloride, lauryl dimethyl benzyl ammonium chloride, lauryl trimethyl ammonium chloride, tris-(oligooxyethyl) alkyl ammonium phosphate, cetyl pyridinium chloride, etc.

Additional examples to so called ester and amido quaternary ammonium compounds are distearyldimonium chloride, dipalmitoylethylhydroxyethylmonium chloride, dioleoylethyl dimethyl ammonium methosulfate, dioleoylethyl hydroxyethylmonium methosulfate, dilinolamidopropyldimonium chloride, dioleylethyl hydroxyethylmonium chloride, dipalmitoylethyldimonium chloride.

From the above quaternary ammonium compounds disclosed with the general formula, especially to mention are those compounds known per se and are on the market, for example, under the trade names "Schercoquat^{®}", "Dehyquart^{®} F30" and "Tetranyl^{®}". Use of these compounds, the so-called "esterquats", in hair care compositions is described, for example, in WO-A 93/107 48, WO-A 92/068 99 and WO-A 94/166 77, wherein, however, there is no reference made to the combinations according to the present invention and the advantageous properties thereof.

Again from the above quaternary ammonium compounds disclosed with the general formula, especially to mention are those compounds are known per se and on the market, for example, under the trade name "INCROQUAT^{®} HO" or "OCS". These compounds are known with a general ingredient category under "amidoquat" in the cosmetic industry.

Compositions mentioned above can also comprise thickening agents to adjust the viscosity to the desired value. All known thickening agents such as anionic, non-ionic, cationic polymers are suitable for the purpose of the invention. It should be noted that compatibility with various ingredients of individual compositions should be paid attention when selecting thickening polymer. Suitable ones are cellulose derivatives such as hydroxyethyl or methyl cellulose, anionic acrylate polymers, cationic cellulose derivatives.

Thickening of the compositions can also be achieved by formulating an emulsion. In such a case at least one fatty alcohol with an alkyl chain length of 12 to 22 C atoms should be present in the composition. Examples are cetyl alcohol, stearyl alcohol or their mixture, myristyl alcohol and behenyl alcohol. Branched fatty alcohols such as octyldodecanol may also be present either alone or in mixture with linear fatty alcohols. Emulsions must also comprise an emulsifier selected from anionic, non-ionic and cationic surfactants as mentioned above. Most preferred emulsifiers are those ethoxylated fatty alcohols as nonionc ones, alkyl sulfates or alkyl ether sulfates as anionc ones and monoalkyl quaternary ammonium ones as cationic surfactants.

Further the above mentioned compositions may comprise additional cationic polymer. Basically suitable are all cationic polymers listed under the generic name "Polyquaternium" in the CTFA International Cosmetic Ingredient Dictionary. Examples are Polyquaternium 6, Polyquaternium 7, Polyquaternium 10, Polyquaternium 11, Polyquaternium 22 and Polyquaternium 28, Polyquaternium 39, Polyquaternium 16 and Polyquaternium 87.

In particular and in further preferred embodiment of the present invention, aqueous reducing composition used in step "c" of the novel process comprises further at least one copolymer of vinylpyrrolidone and quaternized vinylimidazole at a concentration in the range of 0.1 to 2.5% by weight, calculated to total composition. Preferred copolymers of vinylpyrrolidone and quaternized vinylimidazole has a charge density of at least 2 meq/g, preferably 3.0 meq/g, more preferably 6.1 meq/g at pH 7.0. Such polymers are available under the trade name Luviquat from BASF.

The cationic polymers also include the quaternized products of graft polymers from organopolysiloxanes and polyethyl oxazolines described in EP-A 524 612 and EP-A 640 643. Such polymer is known with its CTFA name Polysilicone-9.

Concentration of one or more additional cationic polymers is in the range from 0.05 % to 2.5 %, preferably 0.1 % to 1.5 % by weight, calculated to total composition.

Further the above mentioned composition comprise preferably at least one organic solvent. Suitable organic solvents are 2-methyl-1,3-propanediol, mono and dialcohols or the ethers thereof, in particular mono-C₁-C₃-alkyl ether, ethanol, n-propanol, isopropyl alcohol, 1-methoxypropanol, 1-ethoxypropanol and ethoxydiglycol, diols and their esters 1,3- and 1,4-butanediol, diethyleneglycol and the monomethyl and monoethyl ether thereof, dipropylene glycol and the monomethyl and monoethyl ether thereof, glycerol, hexanetriol, ethyl carbitol, benzyl alcohol, benzyloxy ethanol, propylene carbonate, N-alkyl pyrrolidone, and urea or their mixture preferably in an amount from about 0.1 % to 10 % by weight, calculated to the total composition.

The above mentioned compositions can comprise further ceramide type of compound such as cetyl-PG-hydroxyethylpalmitamide.

Further optional ingredient are sterols, especially the phytosterols are useful hair restructuring compounds can be present in the above mentioned compositions. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

Optionally fatty acids of C10 to C22 may be incorporated into the compositions of the present invention at a concentration of preferably 0.01 to 10%, preferably 0.1 to 5 and more preferably 0.2 to 2.5% by weight calculated to total composition.

In a further preferred embodiment of the present invention, intermediate treatment composition comprises at least one diamine compound. Preferred diamide compounds are according to the general structure 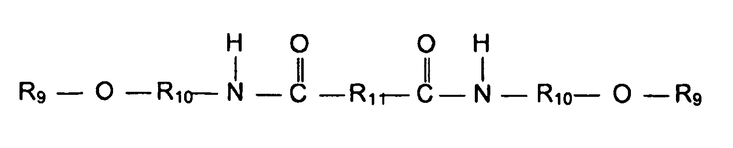 wherein R₉ is a linear or branched, saturated or unsaturated alkyl chain with 1 to 12 C atoms which may be substituted with hydroxy and/or alkoxy groups, preferably R₉ is linear or branched, saturated or unsaturated alkyl chain with 1 to 12 C atoms which may be substituted by 1 to 3 substituents selected from a hydroxy group and C1 to C6 alkoxy group, more preferably R₉ is a unsubstituted alkyl group with 1 to 12 C atoms, and alkyl group with 2 to 12 C atoms substituted by one or two hydroxyl groups, by one alkoxy group with 1 to 6 C atoms or by one hydroxyl and one alkoxy group with 2 to 6 C atoms, R₁₀ is linear or branched alkyl chain with 1 to 5 C atoms, preferably linear or branched alkyl chain with 2 to 5 C atoms and more preferably an alkyl chain with 2 to 3 C atoms, and R₁₁ linear or branched, saturated or unsaturated alkyl chain with 1 to 22 C atoms, preferably linear or branched, saturated or unsaturated alkyl chain with 11 to 22 C atoms.

Preferred individual diamide compounds are the ones according to the formula A to G.

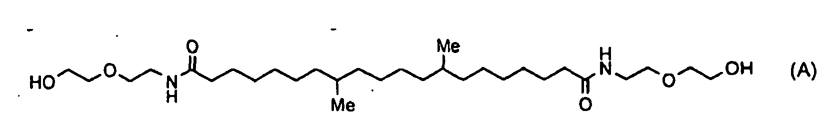

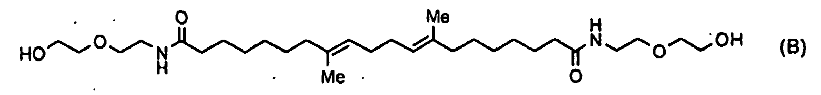

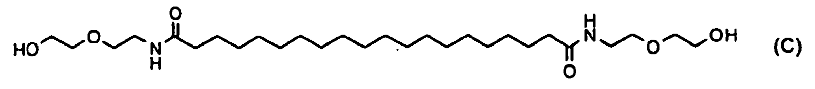

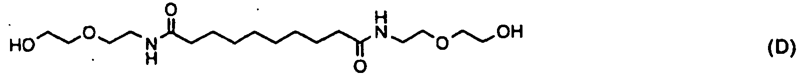

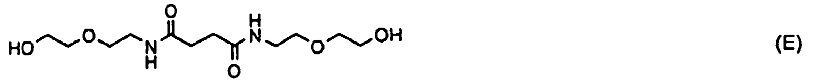

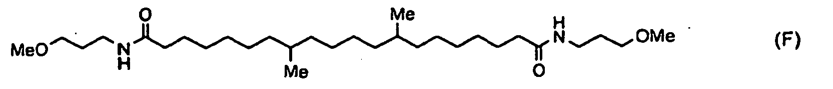

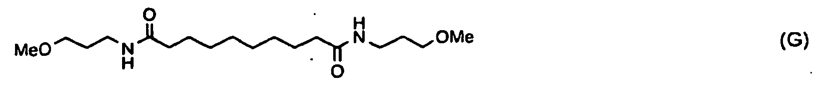

Particularly preferred diamide compound is the compound F which is bis (methoxypropylamido) isodocosane and commercially available from Kao Corporation - Japan.

Concentration of diamide compounds in the intermediate treatment compositions of the present invention is in the range of 0.001 to 5%, preferably 0.002 to 3% more preferably 0.005 to 2% and most preferably 0.01 to 1% by weight calculated to total of each composition.

It is the preferred embodiment of the present invention that oxidative colouring compositions used in steps "a" and "e" of the novel process comprise at least one saturated or unsaturated fatty acid with 12 to 22 C atoms in its molecule at a concentration of 0.1 to 10%, preferably 0.1 to 5% by weight, calculated to total composition.

Another preferred compound in the compositions mentioned above used in the novel process of the present invention is silicone compounds and especially aminated silicones such as amodimethicone available from for example Dow Corning under the brand names Dow Corning 949 Emulsion and Dow Corning 2-8194 ME. Concentration of silicones, especially amodimethicone, is in the range of 0.05 to 2.5%, preferably 0.1 to 1% by weight calculated to total composition.

Additionally, one or more natural oil component may be incorporated into the above mentioned compositions. Suitable are such as olive oil, almond oil, avocado oil, wheatgerm oil, ricinus oil or their mixture. Concentration of these natural oil ingredients should be 0.01 to 2.5%, preferably 0.01.to 1%, more preferably 0.05 to 0.5% by weight, calculated to total composition.

Further additional compounds may be present in the above mentioned compositions of the present invention is ubichinone of the formula 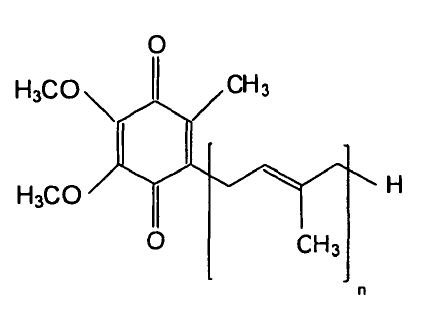 where n is a number between 1 and 10. Preferred ubichinones are the ones where n is a number between 6 and 10 and especially preferred is Ubichinone 50 where n is 10, also known as Coenzyme Q10. Concentration ubichinone of the above formula in the compositions is from 0.0001 to 1%, preferably from 0.0002 to 0.75%, more preferably from 0.0002 to 0.5% and most preferably from 0.0005 to 0.5% by weight, calculated to total composition.

The compositions used according to the invention can naturally comprise all substances customarily found is such compositions such as fragrance, chelating agent, reducing agent in the oxidative colouring compositions for stabilizing oxidation dyes during storage, preservatives, acids or alkaline compounds used for adjusting pH, foam preventing agent such as silicones and especially simethicone.

The following examples are to illustrate but not to limit the invention.

### Example 1

**Oxidative colouring composition for step "a"**

| | |
|---|---|
| Stearyl alcohol | 12.0 (% by wt.) |
| Stearamide MEA | 4.0 |
| Cocamide MEA | 2.0 |
| Propylene glycol stearate SE | 4.0 |
| Sodium lauryl sulfate | 0.3 |
| Oleic acid | 2.0 |
| 1,2-Propanediol | 1.5 |
| Na-EDTA | 0.5 |
| Sodium sulfite | 1.0 |
| Ammonium bicarbonate | 0.95 |
| Ascorbic acid | 0.2 |
| Perfume | 0.4 |
| Ammonia, 25% | 1.0 |
| Ammonium chloride | 0.5 |
| Polysilicone-9 | 0.15 |
| Coenzyme Q10 | 0.001 |
| 2,5-diaminotoluene sulphate | 2.6 |
| 2-amino-4-hydroxyethylaminoanisol sulfate | 1.3 |
| 3-Aminophenol | 0.3 |
| Resorcinol | 0.2 |
| 1-naphtol | 0.15 |
| 4-amino-m-cresol | 0.05 |
| Water | ad 100.00 |

The pH of the above composition is approximately 10.5.

**Oxidizing agent for step "a"**

| | % by weight |
|---|---|
| Hydrogen peroxide | 6% |
| Phosphoric acid | q.s. to pH 2.5 |
| Simethicone | 0.1 |
| Acrylate copolymer | 0.25 |
| Water | to 100 |

The above compositions were mixed at a weight ratio of 1:1 (oxidative dye comprising composition and oxidizing composition) and the mixture had a pH of 6.8.

**Reducing composition for step "c"**

| | % by weight |
|---|---|
| Cetearyl alcohol | 4.5 |
| Steareth-2 | 2.0 |
| Behentrimonium chloride | 1.5 |
| PEG-9 dimethicone | 0.5 |
| Mineral oil | 1.0 |
| Isopropyl palmitate | 1.0 |
| Ammonium thioglycolate (70%) | 3.2 |
| Ammonium hydrogen carbonate | 2.0 |
| Amodimethicone | 0.5 |
| PEG-40-Hydrogenated castor oil | 0.7 |
| Perfume | 0.4 |
| Ethanolamine | 2.3 |
| EDTA | 0.2 |
| Water | ad 100.0 |

The composition has a viscosity of approximately 18,000 mPa.s measured with Brookfield viscosimetre with Spindle 5 at 10 rpm. pH of the composition was 8.3.

**Composition of step "d"**

| | % by weight |
|---|---|
| Magnesium sulphate | 10.0 |
| Asparagic acid | 0.25 |
| Alanin | 0.5 |
| Cetrimonium chloride | 1.0 |
| Hydroxyethylcellulose | 0.25 |
| Citric acid | q.s. to pH 4.10 |
| Water | q.s. to 100 |

**Oxidizing agent for step "e"**

| | % by weight |
|---|---|
| Hydrogen peroxide | 2.5% |
| Phosphoric acid | q.s. to pH 3.5 |
| Acrylate copolymer | 0.25 |
| Water | to 100 |

**Oxidative colouring composition for step "f"**

| | |
|---|---|
| Stearyl alcohol | 12.0 (% by wt.) |
| Stearamide MEA | 4.0 |
| Cocamide MEA | 2.0 |
| Propylene glycol stearate SE | 4.0 |
| Sodium lauryl sulfate | 0.3 |
| Oleic acid | 2.0 |
| 1,2-Propanediol | 1.5 |
| Na-EDTA | 0.5 |
| Sodium sulfite | 1.0 |
| Ammonium bicarbonate | 0.95 |
| Ascorbic acid | 0.2 |
| Perfume | 0.4 |
| Ammonia, 25% | 8.0 |
| Ammonium chloride | 0.5 |
| Polysilicone-9 | 0.15 |
| Coenzyme Q10 | 0.001 |
| 2,5-diaminotoluene sulphate | 3.2 |
| 2-amino-4-hydroxyethylaminoanisol sulfate | 0.8 |
| Resorcinol | 0.8 |
| m-Aminophenol | 0.45 |
| Water | ad 100.00 |

The pH of the above composition is approximately 10.5.

Hair of a female volunteer having approximately shoulder length natural curly hair was coloured and straightened using above compositions. The process was carried out as follows - as described as the first process above and in claim 1.

First of all, oxidative colouring composition of step "a" was applied onto hair after mixing with oxidizing composition for step "a", pH of the colouring composition after mixing was 6.8 and processed for 20 min at approximately 40°C and rinsed off from hair with water and the hair was towel dried. Afterwards, reducing composition of step "c" was applied onto hair and hair was fixed in the straight form on an aluminium sheet surface and processed for approximately 20 min at ambient temperature and rinsed off with water. Subsequently, composition of step "d" was applied and processed for 10 min and without rinsing off oxidizing composition of step "e" was applied onto hair. After processing of additional 10 min oxidative colouring composition of step "f" was applied onto hair. At this step, as an oxidizing agent the same composition used in step "a" was used and the mixture had a pH of approximately 9.5. After processing approximately 30 min at 40°C hair was rinsed off with water and dried.

It was observed that hair was excellently straightened and had an intensive shiny natural black colour.

### Example 2

The example 1 as given above was repeated with the same compositions except the same colouring mixture as in step "a" was also used in step "f" and step "d" was skipped (not used). In other words the colouring mixture used in step "f" had a pH of 6.8, same as in step "e".

Here again intensive shiny natural black colour was obtained and hair had excellent straightened appearance.

### Example 3

The dyestuff composition of the colouring mass was replaced with the following dyestuffs. In this example colouring mass of step "f" of Example 1 (excluding dyestuffs) was used also in step "a".

**Dyestuff composition used in step "a"**

| | % by weight |
|---|---|
| p-toluenediamine sulphate | 0.3 |
| 4-amino-m-cresol | 0.2 |
| 2-amino-3-hydroxypyridine | 0.15 |
| m-aminophenol | 0.1 |
| Resorcinol | 0.07 |

**Dyestuff composition for step "e"**

| | % by weight |
|---|---|
| 4-amino-m-cresol | 0.35 |
| 4-amino-2-hydroxytoluene | 0.2 |
| 2- Hydroxyethyl-p-phenylenediamine sulfate | 0.15 |
| m-aminophenol | 0.1 |
| Resorcinol | 0.2 |
| 2,5,6-triamino-4-pyrimidinol sulphate | 0.05 |
| 2-amino-6-chloro-4-nitrophenol | 0.11 |

A shoulder length medium blond curled hair was coloured using the colouring composition used in example 1 step "f" with the above dyestuffs.

First of all, colouring composition was mixed with oxidizing agent (example 1 step "a") at a weight ratio of 2:1 and applied onto hair. The composition mixed had a pH of 9.5. Processing time was 20 min and afterwards hair was rinsed off and towel dried. Afterwards, reducing composition of step "c" was applied onto hair and hair was combed through with an appropriate comb during processing time of approximately 20 min at ambient temperature and rinsed off with water. Subsequently, oxidizing composition of step "e" was applied onto hair. After processing of10 min without rinsing off oxidative colouring composition of step "f" was applied onto hair. At this step, as an oxidizing agent the same composition used in step "a" was used and the mixture had a pH of approximately 9.5. After processing approximately 30 min at 40°C hair was rinsed off with water and dried.

It was observed that hair had excellent elasticity and straightened appearance and as well as had an intensive shiny blonde copper tone.

### Example 4

In this example oxidative colouring composition used in example 3 was used in step "a". The colouring composition of step "f" was same as step "a" of example 1 except the dyestuffs which was as follows.

**Dyestuff composition for step "e"**

| | % by weight |
|---|---|
| 2-amino-3-hydroxypyridine | 0.5 |
| p-toluenediamine sulphate | 0.35 |
| 4-amino-m-cresol | 0.3 |
| Resorcinol | 0.1 |
| 2,5,6-triamino-4-pyrimidinol sulphate | 0.1 |
| Sodium picramate | 0.15 |
| HC Red No 3 | 0.35 |
| HC Yellow No 2 | 0.35 |

**Composition for step "d"**

| | % by weight |
|---|---|
| Dimethicone | 25 |
| Phenyltrimethicone | 1 |
| Avocado oil | 1 |
| Isopropylpalmitate | 1 |
| Cetrimonium chloride | 0.2 |
| Amodimethicone | 0.5 |
| Citric acid | q.s. to pH 5.0 |
| Water | q.s. to 100 |

The above composition when prepared by mixing all the components is a two phase composition and must be shaken to homogeneity before application onto hair.

A shoulder length medium blond naturally curled hair was coloured and straightened using the colouring composition used in example 1 step "f" with the above dyestuffs.

First of all, colouring composition was mixed with oxidizing agent (example 3 step "a") at a weight ratio of 2:1 and applied onto hair. The composition mixed had a pH of 9.5. Processing time was 20 min and afterwards hair was rinsed off and towel dried. Afterwards, reducing composition of step "c" was applied onto hair and processed for approximately 20 min at ambient temperature and rinsed off with water and hair was dried with a hair drier. Subsequently, composition of step "d" was applied, afterwards, a hot iron suitable for straightening hair and commercially available was used to straighten hair at a temperature of approximately 140°C. The hot iron has a round shaped ironing metal part. It was also observed in an additional trial a flat shaped hot iron can be used in the same way. This was followed by application of oxidizing composition of step "e" and processing of 10 min. Oxidative colouring composition of step "f" was applied onto hair afterwards. At this step, as an oxidizing agent the same composition used in step "a" was used and the mixture had a pH of approximately 9.5. After processing approximately 30 min at 40°C hair was rinsed off with water and dried.

It was observed that hair had excellent elasticity and curl appearance and as well as had an intensive shiny dark blonde copper tone.

## Claims

1. Process for colouring and straightening hair in a single process **characterised in that** it comprises the following steps
a- an aqueous oxidative colouring composition based on at least one oxidative dye precursor and optionally at least one coupling substance but not comprising any direct dye is mixed with a composition comprising at least one oxidizing agent and applied onto hair and rinsed off from hair with water after processing 5 to 45 min at a temperature of 20 to 45°C, optionally shampooed,
b- towel dried,
c- an aqueous composition comprising at least one reducing agent is applied onto hair and rinsed off from hair with water after a processing time of 2 to 30 min at ambient temperature wherein hair is continuously combed through during processing with reducing agent and towel dried,
d- optionally hair is treated with a composition comprising at least one salt and optionally rinsed off,
e- optionally a composition comprising at least one oxidizing agent is applied onto hair processed for 2 to 15 min and optionally rinsed off from hair with water and optionally towel dried, and
f- an aqueous oxidative colouring composition based on at least one oxidative dye precursor and optionally at least one coupling substance is mixed with a composition comprising at least one oxidizing agent and applied onto hair and rinsed off from hair with water after processing 5 to 45 min at a temperature of 20 to 45°C and towel dried and dried with a dryer or left to dry in the air.

2. Process according to claim 1 **characterised in that** the oxidative colouring composition has pH between 5 and 12 after mixing with a composition comprising at least one oxidizing agent.

3. Process according to claims 1 and 2 **characterised in that** reducing composition of step "c" comprises at least one reducing agent selected from thioglycolic acid, thiolactic acid and/or their salts in particular ammonium and ethanolamine salts, cystein and/or hydrochloride salt thereof, homocysteine, cysteamine, N-acetyl cysteine, thioglycerol, ethanediol monothioglycolate, 1,2-propyleneglycol monothioglycolate, 1,3-propanediol monothioglycolate or the isomer mixture resulting therefrom, 1,4-butanediol monothioglycolate and the isomer mixtures therefrom, polyethylene glycol, such as di-, tri- and tetraethyleneglycol monothioglycolates, glycerol monothiolactate and further thio acids and the esters thereof, as well as mixtures thereof at a concentration of 2 to 15%, by weight, calculated to total composition.

4. Process according to any of the preceding claims **characterised in that** reducing composition at step "c" has a pH between 6.5 and 10.5.

5. Process according to any of the preceding claims **characterised in that** the composition used in step "d" comprises at least one oil selected from natural oils, fatty acid fatty alcohol esters and silicone oils.

6. Process according to any of the preceding claims **characterised in that** composition used at step "d" comprises at least one volatile silicone oil and at least one non-volatile silicone oil.

7. Process according to any of the preceding claims **characterised in that** the oxidizing agent used in step "e" comprises at least one oxidizing agent at a concentration 1 to 5% by weight, calculated to total composition and has a pH from 2 to 6.

8. Process according to any of the preceding claims **characterised in that** oxidative colouring composition of step "f' comprises at least one direct dye.

9. Process according to any of the preceding claims **characterised in that** any of the compositions used in any step of the process comprises a thickening agent selected from anionic, non-ionic and cationic polymers.

10. Process according to any of the preceding claims **characterised in that** any of the compositions used in any step of the process is an emulsion and comprises at least fatty alcohol with an alkyl chain length of 12 to 22 C atoms.

11. Process according to any of the preceding claims **characterised in that** any of the compositions used in any step of the process comprises a surfactant selected from anionic, cationic, non-ionic and amphoteric ones, wherein cationic surfactant is preferably selected from compounds of formula 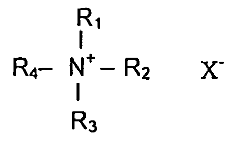 where R₁ is a saturated or unsaturated, branched or non-branched alkyl chain with 8-22 C atoms or
R₅CO NH (CH₂)n
where R₅ is saturated or unsaturated, branched or non-branched alkyl chain with 7-21 C atoms and n has typical value of 0 - 4 or
R₆CO O (CH₂)n
where R₆ is saturated or unsaturated, branched or non-branched alkyl chain with 7-21 C atoms and n has typical value of 0 - 4, and
R₂ is a hydrogen, saturated or unsaturated, branched or non-branched alkyl chain with 1-22 C atoms or
R₅CO NH (CH₂)ₙ
where R₅ is saturated or unsaturated, branched or non-branched alkyl chain with 7-21 C atoms and n has typical value of 0 - 4 or
R₆ CO O (CH₂)n
where R₆ is saturated or unsaturated, branched or non-branched alkyl chain with 7-21 C atoms and n has typical value of 0 - 4,
and R₃ and R₄ are independent from each other H or lower alkyl chain with 1 to 4 carbon atoms, and X is chloride, bromide or methosulfate.

12. Process according to any of the preceding claims **characterised in that** any of the compositions used in any step of the process comprises one or more of compounds selected from organic solvent, silicone compound, fatty acid with 12 to 22 C atoms, cationic polymer and ubichinone of the formula 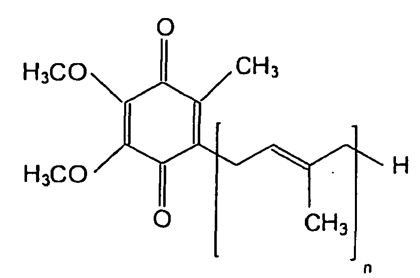 where n is a number between 1 and 10.

## Patentansprüche

1. Verfahren zum Färben und Glätten von Haaren in einem Prozess, **dadurch gekennzeichnet, dass** es die folgenden Schritte beinhaltet :
a- eine wässrige oxidative Farbzusammensetzung, basierend auf mindestens einem oxidativen Farbentwickler und optional mindestens einer Kupplersubstanz, die aber keinerlei direkt ziehende Farbstoffe enthält, wird mit einer Zusammensetzung, die mindestens eine oxidierende Verbindung enthält gemischt und auf das Haar aufgetragen und nach einer Einwirkzeit von 5 bis 45 Minuten bei einer Temperatur von 20 bis 45 °C aus dem Haar gespült, optional shampooniert,
b- Handtuch getrocknet,
c- eine wässrige Zusammensetzung, die mindestens eine reduzierende Verbindung enthält wird auf das Haar aufgetragen und nach einer Einwirkzeit von 2 bis 30 Minuten bei Raumtemperatur mit Wasser ausgespült und mit dem Handtuch getrocknet, wobei das Haar während der Einwirkzeit mit dem Reduktionsmittel kontinuierlich durchgekämmt wird,
d- optional wird das Haar mit einer Zusammensetzung die mindestens ein Salz enthält behandelt und optional ausgespült,
e- optional wird eine Zusammensetzung die mindestens eine oxidierende Verbindung enthält auf das Haar aufgetragen und nach einer Einwirkzeit von 2 bis 15 Minuten optional aus dem Haar mit Wasser ausgespült und optional mit dem Handtuch getrocknet, und
f- eine wässrige oxidative Farbzusammensetzung basierend auf mindestens einem oxidativen Farbentwickler und optional mindestens einer Kupplersubstanz wird mit einer Zusammensetzung die mindestens ein Oxidationsmittel enthält gemischt und nach einer Einwirkzeit von 5 bis 45 Minuten bei einer Temperatur von 20 bis 45°C mit Wasser ausgespült und mit dem Handtuch getrocknet, mit einem Fön getrocknet oder Luftgetrocknet.

2. Verfahren nach Anspruch 1, **dadurch gekennzeichnet, dass** die oxidative Farbzusammensetzung nach dem Mischen mit einer Zusammensetzung die mindestens ein Oxydationsmittel enthält einen pH- Wert zwischen 5 und 12 hat.

3. Verfahren nach den Ansprüchen 1 und 2, **dadurch gekennzeichnet, dass** die reduzierende Zusammensetzung von Schritt "c" mindestens ein Reduktionsmittel, ausgewählt aus Thioglykolsäure, Thiomilchsäure und/oder deren Salze im speziellen Ammonium- und Ethanolaminsalze, Cystein und/oder Hydrochloridsalz davon, Homocysteine, Cysteamine, N-Acetyl cysteine, Thioglycerol, Ethanediolmonothioglycolate, 1,2-Propyleneglycol monothioglycolate, 1,3-Propandiolmonothioglycolate oder die daraus resultierende Isomermischung 1,4-Butanediolmonothioglycolat und deren Isomermischung, Polyethylenglycol, wie Di-, Tri- und Tetraethyleneglycolmonothioglycolate, Glycerolmonothiolactat und weitere Thiosäuren und deren Ester, als auch Mischungen davon in einer Menge von 2 bis 15 Gew.- % enthält, berechnet auf die Gesamtzusammensetzung.

4. Verfahren nach einem der vorhergehenden Ansprüche, **dadurch gekennzeichnet, dass** das Reduktionsmittel von Schritt "c" einen pH zwischen 6,5 und 10,5 hat.

5. Verfahren nach einem der vorhergehenden Ansprüche, **dadurch gekennzeichnet, dass** die Zusammensetzung die in Schritt "d" verwendet wird mindestens ein Öl, ausgewählt aus natürlichen Ölen, Fettsäure-Fettalkoholestern und Silikonölen enthält.

6. Verfahren nach einem der vorhergehenden Ansprüche, **dadurch gekennzeichnet, dass** die Zusammensetzung die in Schritt "d" verwendet wird, mindestens ein flüchtiges Silikonöl und mindestens ein nicht flüchtiges Silikonöl enthält.

7. Verfahren nach einem der vorhergehenden Ansprüche, **dadurch gekennzeichnet, dass** das Oxydationsmittel, das in Schritt "e" verwendet wird mindestens ein Oxydationsmittel in einer Menge von 1 bis 5 Gew.- % enthält, berechnet auf die Gesamtzusammensetzung und einen pH von 2 bis 6 hat.

8. Verfahren nach einem der vorhergehenden Ansprüche, **dadurch gekennzeichnet, dass** die oxidative Farbzusammensetzung von Schritt "f" mindestens eine direkt ziehende Farbe enthält.

9. Verfahren nach einem der vorhergehenden Ansprüche, **dadurch gekennzeichnet, dass** jede der Zusamensetzungen die in einem der Schritte des Verfahrens verwendet wird ein Verdickungsmittel, ausgewählt aus anionischen, nicht ionischen und kationischen Polymeren, enthält.

10. Verfahren nach einem der vorhergehenden Ansprüche, **dadurch gekennzeichnet, dass** jede der Zusammensetzungen die in einem der aufgeführten Schritte des Verfahrens verwendet wird eine Emulsion ist und mindestens einen Fettalkohol mit einer Alkylkettenlänge von 12 bis22 C- Atomen enthält.

11. Verfahren nach einem der vorhergehenden Ansprüche, **dadurch gekennzeichnet, dass** jede der Zusammensetzung die in einem der aufgeführten Schritte des Verfahrens verwendet wird ein Tensid enthält, ausgewählt aus anionischen, kationischen, nicht ionischen und amphoterischen Tensiden, wobei das kationische Tensid vorzugsweise ausgewählt ist aus Verbindungen der Formel 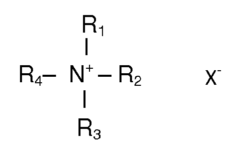 worin R₁ eine gesättigte oder ungesättigte, verzweigte oder nicht verzweigte Alkylkette mit 8 - 22 C- Atomen ist oder
R₅CO NH (CH₂)n
worin R₅ eine gesättigte oder ungesättigte, verzweigte oder nicht verzweigte Alkylkette mit 7 - 21 C- Atomen ist und n einen typischen Wert von 0 - 4 hat oder
R₆CO O (CH₂)n
worin R₆ ein gesättigte oder ungesättigte, verzweigte oder nicht verzweigte Alkylkette mit 7 - 21 C- Atomen ist und n einen typischen Wert von 0 - 4 hat und
R₂ ein Wasserstoff, eine gesättigte oder ungesättigte, verzweigte oder nicht verzweigte Alkylkette mit 1 - 22 C- Atomen ist oder
R₅CO NH (CH₂)n
worin R₅ eine gesättigte oder ungesättigte, verzweigt oder nicht verzweigte Alkylkette mit 7 - 21 C- Atomen ist und n einen typischen Wert vo 0 - 4 hat oder
R₆ CO O (CH₂)n
worin R₆ eine gesättigte oder ungesättigte, verzweigte oder nicht verzweigte Alkylkette mit 7 - 21 C- Atomen ist und n einen typischen Wert von 0 - 4 hat, und
R₃ und R₄ unabhängig voneinander Wasserstoff oder niedrige Alkylketen mit 1 bis 4 Kohlenstoffatomen sind, und X Chlorid, Bromid oder Methosulfat ist.

12. Verfahren nach einem der vorhergehenden Ansprüche, **dadurch gekennzeichnet, dass** jede Zusammensetzung die in einem der beschriebenen Schritte des Verfahrens verwendet wird eine oder mehrere Verbindungen, ausgewählt aus organischen Lösungsmitteln, Silikonverbindungen, Fettsäure mit 12 bis 22 C- Atomen, kationisches Polymer und Ubichinon der Formel 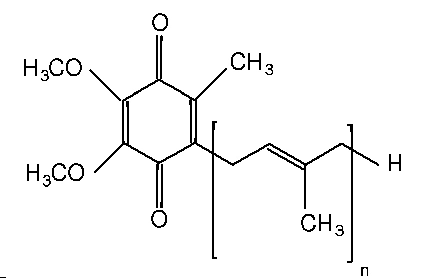 enthält, worin n eine Zahl zwischen 1 und 10 ist.

## Revendications

1. Procédé de coloration et de lissage des cheveux en un procédé unique **caractérisé par** les étapes suivantes
a- une composition de coloration d'oxydation aqueuse basée sur au moins un précurseur de colorant oxydant et, facultativement, au moins une substance de couplage mais ne comprenant pas de colorant direct, est mélangée avec une composition comprenant au moins un agent oxydant et appliquée sur la chevelure puis rincée à l'eau après avoir laissé agir 5 à 45 min à une température de 20 à 45°C, shampooing facultatif,
b- séchage avec une serviette
c- une composition aqueuse comprenant au moins un agent réducteur est appliquée sur la chevelure et rincée à l'eau après un temps de pose de 2 à 30 min à température ambiante, alors que la chevelure est peignée continuellement durant le traitement avec un agent réducteur puis séchée avec une serviette,
d- facultativement, la chevelure est traitée avec une composition comprenant au moins un sel et est facultativement rincée,
e- facultativement, une composition comprenant au moins un agent oxydant est appliquée sur la chevelure traitée pendant 2 à 15 min et est facultativement rincée à l'eau et facultativement séchée avec une serviette, et
f- une composition de coloration d'oxydation aqueuse basée sur au moins un précurseur de colorant oxydant et, facultativement, au moins une substance de couplage est mélangée avec une composition comprenant au moins un agent oxydant et appliquée sur la chevelure et rincée à l'eau après avoir laissé agir 5 à 45 min à une température de 20 à 45°C, la chevelure est ensuite séchée avec une serviette et séchée avec un sèche-cheveux ou laissée sécher à l'air libre.

2. Le procédé selon la revendication 1, **caractérisé par le fait que** la composition de coloration d'oxydation a un pH compris entre 5 et 12 après avoir été mélangée avec une composition comprenant au moins un agent oxydant.

3. Le procédé selon les revendications 1 et 2, **caractérisé par le fait que** la composition réductrice de l'étape "c" comprend au moins un agent réducteur choisi parmi l'acide thioglycolique, l'acide thiolactique et/ou leurs sels, en particulier les sels d'ammonium et d'éthanolamine, cystéine et/ou les sels hydrochlorures de celle-ci, l'homocystéine, la cystéamine, la N-acétylcystéine, le thioglycérol, le monothioglycolate d'éthylène glycol, le monothioglycolate de 1,2-propylène glycol, le monothioglycolate de 1,3-propanediol ou le mélange isomère en résultant, le monothioglycolate de 1,4-butanediol et les mélanges isomères en résultant, le polyéthylène glycol tel que les monothioglycolates de di-, tri- et tetraéthylèneglycol, le monothioglycolate de glycérol et d'autres acides thio et leurs esters, ainsi que leurs mélanges à une concentration de 2 à 15% en poids par rapport au poids total de la composition.

4. Le procédé selon l'une des revendications précédentes quelconque, **caractérisé par le fait que** la composition réductrice de l'étape "c" a un pH compris entre 6,5 et 10,5.

5. Le procédé selon l'une des revendications précédentes quelconque, **caractérisé par le fait que** la composition utilisée dans l'étape "d" comprend au moins une huile choisie parmi les huiles naturelles, les acides gras, les esters d'alcool gras et les huiles silicones.

6. Le procédé selon l'une des revendications précédentes quelconque, **caractérisé par le fait que** la composition utilisée dans l'étape "d" comprend au moins une huile silicone volatile et au moins une huile silicone non-volatile.

7. Le procédé selon l'une des revendications précédentes quelconque, **caractérisé par le fait que** l'agent oxydant utilisé dans l'étape "e" comprend au moins un agent oxydant à une concentration de 1 à 5% en poids par rapport au poids total de la composition et a un pH entre 2 et 6.

8. Le procédé selon l'une des revendications précédentes quelconque, **caractérisé par le fait que** la composition de coloration d'oxydation de l'étape "f" comprend au moins un colorant direct.

9. Le procédé selon l'une des revendications précédentes quelconque, **caractérisé par le fait que** toutes les compositions utilisées dans toutes les étapes du traitement comprennent un agent épaississant choisi parmi les polymères anioniques, cationiques et non-ioniques.

10. Le procédé selon l'une des revendications précédentes quelconque, **caractérisé par le fait que** toutes les compositions utilisées dans toutes les étapes du traitement sont une émulsion et comprennent au moins un alcool gras avec une chaîne alcane d'une longueur allant de 12 à 22 atomes de carbone.

11. Le procédé selon l'une des revendications précédentes quelconque, **caractérisé par le fait que** toutes les compositions utilisées dans toutes les étapes du traitement comprennent un agent tensioactif choisi parmi les agents tensioactifs anioniques, cationiques, non-ioniques et amphotères, où l'agent tensioactif cationique est choisi de préférence parmi les composés de formule suivante : 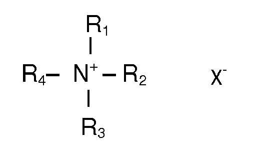 où R₁ est une chaîne alcane saturée ou insaturée, ramifiée ou non-ramifiée avec 8-22 atomes C ou
R₅CO NH (CH₂)n
où R₅ est une chaîne alcane saturée ou insaturée, ramifiée ou non-ramifiée avec 7-21 atomes C et n a une valeur typique de 0 - 4 ou
R₆CO O (CH₂)n
où R₆ est une chaîne alcane saturée ou insaturée, ramifiée ou non-ramifiée avec 7-21 atomes C et n a une valeur typique de 0 - 4 et
R₂ est un atome d'hydrogène ou une chaîne alcane saturée ou insaturée, ramifiée ou non-ramifiée avec 1-22 atomes C ou
R₅CO NH (CH₂)n
où R₅ est une chaîne alcane saturée ou insaturée, ramifiée ou non-ramifiée avec 7-21 atomes C et n a une valeur typique de 0 - 4 ou
R₆ CO O (CH₂)n
où R6 est une chaîne alcane saturée ou insaturée, ramifiée ou non-ramifiée avec 7-21 atomes C et n a une valeur typique de 0 - 4,
et R₃ et R₄, indépendants l'un de l'autre, sont un atome H ou une chaîne alcane inférieure avec 1 - 4 atomes de carbones, et X est un atome de chlorure, bromure ou méthosulfate.

12. Le procédé selon l'une des revendications précédentes quelconque, **caractérisé par le fait que** toutes les compositions utilisées dans toutes les étapes comprennent au moins un des composés choisis parmi les : solvants organiques, composés siliconés, acides gras avec 12 à 22 atomes de carbone, polymères cationiques et ubiquinone de formule suivante : 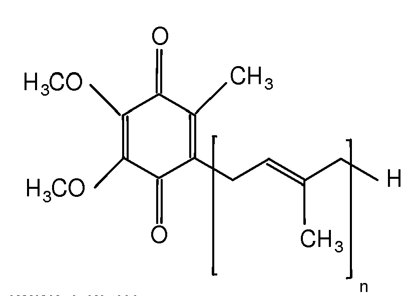 où n est un nombre entre 1 et 10
